# EUROPÄISCHE PATENTSCHRIFT

(11) **EP 2 922 506 B1**
(45) Veröffentlichungstag und Bekanntmachung des Hinweises auf die Patenterteilung: **29.03.2017**
(21) Anmeldenummer: 13821716.1
(22) Anmeldetag: 26.11.2013
(51) Int. Cl.: A61F 5/01

(54) **MODULARES ORTHESENSYSTEM UND KIT ZU DESSEN ANPASSUNG**
MODULAR ORTHOSIS SYSTEM AND KIT FOR THE FITTING THEREOF
SYSTÈME D'ORTHÈSE MODULAIRE ET KIT PERMETTANT SON ADAPTATION

(30) Priorität: 26.11.2012 DE 102012023028
(43) Veröffentlichungstag der Anmeldung: 30.09.2015
(73) Patentinhaber: Orthopunkt AG, 4500 Solothurn (CH)
(72) Erfinder: SCHRÖDER, Jan-Hagen, CH-4500 Solothurn (CH)
(74) Vertreter: Seuss, Thomas
(86) Internationale Anmeldenummer: PCT/IB2013/002645
(87) Internationale Veröffentlichungsnummer: WO 2014/080274

(56) Entgegenhaltungen:
- WO-A1-2005/117772
- DE-A1-102010 019 844
- DE-B3-102005 058 999
- US-A1- 2008 300 525

## Beschreibung

Die Erfindung betrifft ein modulares Orthesensystem für eine Orthese der unteren Extremität, welches eine individuelle Anpassung an den jeweiligen Patienten und seinen Funktionsverlust erleichtert sowie im Laufe der Therapie an veränderte Situationen schnell angepasst werden kann.

### Hintergrund der Erfindung

Orthesen sind medizinische Hilfsmittel zur Unterstützung von Limitierungen der Funktionsfähigkeit von Extremitäten, beispielsweise als Folge von Cerebralparesen, Fußheberlähmungen, Schlaganfällen, Muskeldystrophien oder Polio Myelitis. Orthesen erlauben die Fixierung von Körperteilen zur Bewegungsstabilisierung und/oder Schonung sowie Bewegungsunterstützung von Gelenken. Orthesen werden äußerlich an die zu therapierende Extremität angelegt und über längere Zeiträume getragen. Da die menschliche Anatomie, wie auch die Art der Bewegungseinschränkung, sehr unterschiedlich sein kann, müssen Orthesen individuell an den jeweiligen Patienten angepasst werden. Aus dem Stand der Technik sind Orthesen bekannt, die durch eine Vielzahl von Einstelleinrichtungen angepasst werden können. Andere Orthesen werden nach genauer Messung der ergonomischen Verhältnisse des Patienten individuell angefertigt, beispielsweise durch Methoden des "rapid prototyping" bzw. "rapid manufacturing". Gleichwohl hat sich herausgestellt, dass es bei vielen Patienten nur schwer vorhersehbar ist, welche genaue Gestaltung der Orthese das Bewegungsverhalten optimal unterstützt.

Bei den Orthesensystemen nach dem Stand der Technik müssen bereits vor Anfertigung der Orthese einzelne Ausführungsformen festgelegt werden:
In der WO 2005/117772 A1, die den nächstliegenden Stand der Technik darstellt, wird eine Orthese mit einem Fußteil und einer Unterschenkelmanschette beschrieben, die mittels eines Federelements verbunden sind und bei der das Federelement ausschließlich anterior (ventral) geführt und dorsal mit dem Fußteil verbunden wird.

In der DE 10 2010 019 844 A1 wird ebenfalls ein modulares Orthesensystem beschrieben, mit einem Fußteil und einer Unterschenkelmanschette beschrieben, die mittels eines Federelements verbunden sind, Bei dem System der DE 10 2010 019 844 A1 wird das Federelement ausschließlich posterior (dorsal) geführt und mit dem Fußteil verbunden wird.

Bei diesen Unterschenkelorthese muss vor der Anfertigung festgelegt werden, ob die Stütze ventral oder dorsal vorhanden sein soll. In der Regel müssen die Härtegrade und Höhe von Sohle und Stütze festgelegt werden, da eine spätere Auswechslung nicht möglich ist. Aufgrund dieser Rahmenbedingungen kann der Patient nicht unterschiedliche Ausführungsformen miteinander vergleichen, so dass nicht feststellbar ist, ob eine bestimmte Orthese bereits die optimale Versorgung darstellt. Darüber hinaus sind die gängigen Orthesensysteme nicht ohne komplette Neuherstellung an körperliche Veränderungen adaptierbar (z.B. an das Größenwachstum der Patienten). In der Regel werden aufgrund des Kostendruckes daher Kompromisslösungen hergestellt.

### Aufgabe der Erfindung

Aufgabe der Erfindung ist es daher, ein modulares Orthesensystem für eine Orthese der unteren Extremität zur Verfügung zu stellen, welches eine individuelle Anpassung an den jeweiligen Patienten und an seinen Funktionsverlust erleichtert sowie im Laufe der Therapie an veränderte Situationen schnell angepasst werden kann.

### Lösung der Aufgabe

Die Lösung der gestellten Aufgabe erfolgt durch ein modulares Orthesensystem gemäß Anspruch 1.

Das erfindungsgemäße Orthesensystem enthält die folgenden Elemente:
eine thermisch ausgeformte Sohle mit einer oder mehreren vorgeformten Aufnahmen für Verriegelungselemente und einer sandalenförmigen Fußmanschette mit Polsterung und Fixiereinrichtungen,
eine Unterschenkelmanschette mit einer oder mehreren Fixiereinrichtungen zur Befestigung am Unterschenkel, einer Polsterung sowie einer Aufnahmevorrichtung für ein oder mehrere Stützfedern,
eine Stützfeder mit einem oberen flachen Teil sowie einem unteren halbrund ausgeformten Teil, wobei das halbrund ausgeformte Teil eine Ausnehmung zur Aufnahme der Sohle ausbildet,
wobei die Stützfeder mit der Unterschenkelmanschette reversibel verbunden ist,
wobei die Stützfeder an der Fußmanschette dorsal/medial, dorsal, lateral, ventral/medial oder ventral/lateral befestigt sein kann.

Die Fußmanschette ist nach Art einer Sandale gefertigt, so dass Sie die Fußsohle komplett abdeckt, aber nach oben weitgehend offen ist. Wenigstens ein Teil der Manschette verläuft über den Fußrücken, wenigstens ein weiterer Teil der Manschette verläuft hinter der Ferse, so dass die Manschette fest am Fuß fixiert werden kann. Die Fußmanschette kann aus jedem Material gefertigt werden, welches ausreichend stabil ist (z.B. Laminate mit Polytol oder Orthocryl weich). Vorzugsweise handelt es sich um ein thermoplastisch verformbares Material, welches kleinere Anpassungen erlaubt. Erfindungsgemäße Fußmanschetten können in Standardfußgrössen gefertigt werden, können aber auch individuell nach einem Gipsabdruck oder mit Unterstützung eines CAD/CAM-Systemes erstellt werden. Verfahren zur Herstellung derartiger Fußmanschetten sind prinzipiell bekannt.

Die Fußmanschette und die Unterschenkelmanschette erhält innen ein Polster, aus waschbarem, wärme- und feuchtigkeitstransportierendem, luftdurchlässigen, klettfähigen 3D-Abstandsgewirke (z.B. Space Tex).
Die Polsterung kann in Kombination mit Microklett einfach entfernt und gewaschen werden.

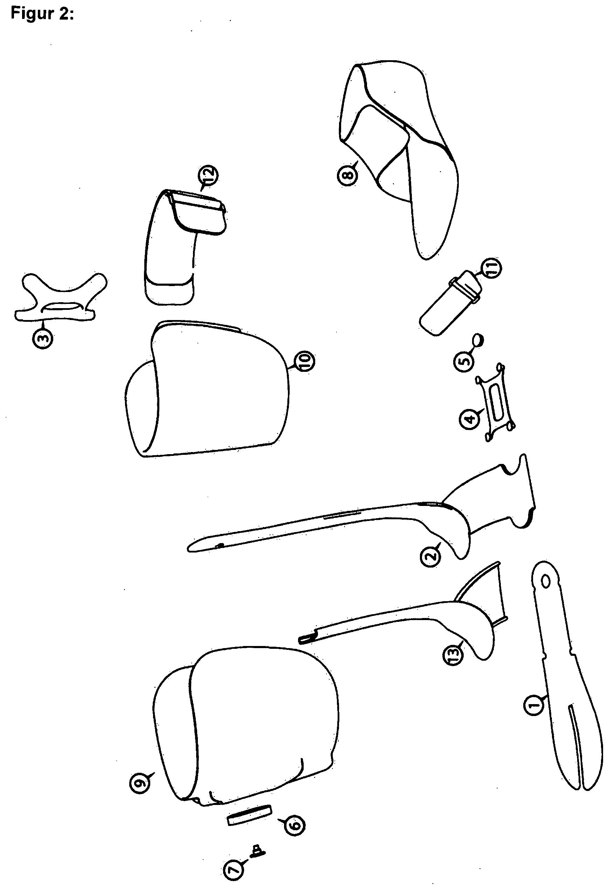

Die Verriegelung der Sohle mit der sandalenförmigen Fußmanschette geschieht durch die Verwendung eines Verriegelungselementes welches sich zentral unter der Ferse des Patienten befindet (siehe Figur 2, Nr. 5). In der bevorzugten Ausführungsform ist dieses Verriegelungselementes elastisch, aber weniger elastisch, als die Polsterung, so dass das Verriegelungselement afferenzverstärkend wirkt. Es hat sich gezeigt, dass Patienten mit den eingangs genannten Funktionseinschränkungen der unteren Extremitäten häufig auch Wahrnehmungsstörungen haben, so dass sie beim Laufen Schwierigkeiten haben wahrzunehmen, wann der Fuß auf den Boden auftrifft, insbesondere bei Verwendung einer Orthese. Diese Wahrnehmungsschwierigkeiten führen zu weiteren Folgeschwierigkeiten im Gangbild des Patienten. Durch Verwendung eines afferenzverstärkenden Verriegelungselementes, welches sich möglichst zentral unter der Ferse des Patienten befindet, wird die Wahrnehmung des Auftretens auf den Boden deutlich verbessert, was zu einer weiteren Verbesserung des Gangbildes des Patienten beiträgt. Es versteht sich von selbst, dass das afferenzverstärkende Verriegelungselement hinsichtlich der Dimensionen und des Elastizitätsgrades so gestaltet sein muss, dass keine Druckläsionen beim Patienten auftreten.

Die Unterschenkelmanschette ist U-förmig ausgebildet und umfasst den Unterschenkel weitgehend. Sie wird mittels einer ergänzenden Fixiereinrichtung (z.B. einer Schnalle oder eines Klettbandes) am Unterschenkel befestigt. Um die verschiedenen Unterschenkelgrößen verschiedener Patienten abzubilden kann die Unterschenkelmanschette in Standardgrößen angeboten werden. Bevorzugt wird sie individuell erstellt, beispielsweise nach einem Gipsabdruck. Die Unterschenkelmanschette ist aus verschiedenen Materialien herstellbar, sofern das Material eine ausreichende Dauerbelastbarkeit aufweist (wie z.B. Laminate mit Polytol oder Orthocryl weich). Bevorzugt handelt es sich um ein thermoplastisches Material, welches kleinere Anpassungen erlaubt. Die Unterschenkelmanschette enthält darüber hinaus ein Polster aus waschbarem, wärme- und feuchtigkeitstransportierendem, luftdurchlässigen, klettfähigen 3D-Abstandsgewirke (z.B. Space Tex).

Ein weiterer wichtiger Aspekt der Erfindung ist die Stütze (Steg).
Diese Stütze (auch als "*dynamic steg*" bezeichnet) ist im Wesentlichen eine Blattfeder (mit energierückgewinnender Push Up Funktion), deren Steifigkeit an das Individuum unter besonderer Berücksichtigung des Körpergewichtes der Körpergrösse sowie an den Muskeltonus angepasst wird. Materialien, Methoden zu Ihrer Anpassung sowie Messverfahren zur Messung der Rückstellkraft sind aus dem Stand der Technik bekannt (siehe z.B Novacheck et al., J. Prosthet Orthot, 2007; 19:98-103). Die Stützfeder wird an ihrem oberen Ende in eine entsprechende Öffnung mit Führung der Unterschenkelmanschette eingeführt und mit dieser durch Verklemmung und Verschraubungssicherung mit einer unidirektional schwimmend gelagerten Mutter, wie es z.B. bei einer Lagerung mit einem Elastomer der Fall ist, fast gelenkartig verbunden. An ihrem unteren Ende ist sie halbrund geformt, um die Fußmanschette zu umschließen. Ergänzende Fixiereinrichtungen bewirken, dass sich die Fußmanschette während des Gebrauchs nicht von der Stützfeder löst. Die Stützfeder kann optional durch ein Schutzelement "Bumper" oberflächlich vor mechanischen Beschädigungen geschützt werden. Dieser Bumper ist bevorzugt aus einem elastischen Material, wie Kautschuk und/oder Silikon gefertigt. Der Bumper kann in unterschiedlichen Farben und Gestaltungen gefertigt werden und/oder bedruckt und/oder anderweitig mit Mustern versehen werden. Neben der Schutzfunktion für das Material der Stützfeder hat der Bumper daher auch eine gestalterische Funktion.

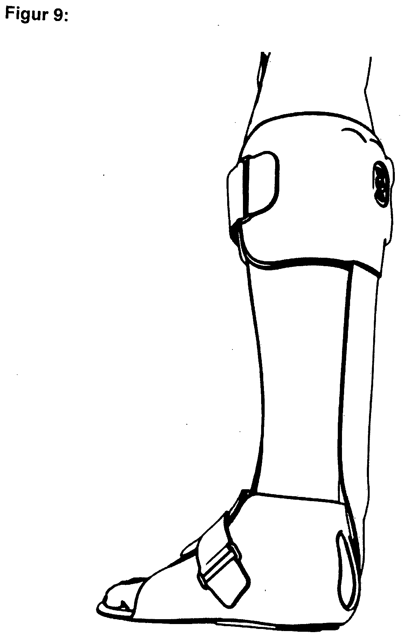

Die Stützfeder wird an ihrem oberen Ende in eine entsprechende Öffnung der Unterschenkelmanschette eingeführt und mit dieser verschraubt. Die Details dieser Verschraubung werden weiter unten offenbart. An ihrem unteren Ende ist sie halbrund geformt um die Fußmanschette zu umschließen. Eine Besonderheit des erfindungsgemäßen Systems ist, dass die Umschließung der Fußmanschette medial oder lateral sein kann. Weiterhin kann die Stützfeder dorsal oder ventral getragen werden. Es ist dem Fachmann klar, dass bei einer Ausführungsänderung der erfindungsgemäßen Orthese von ventraler zu dorsaler Tragweise die Unterschenkelmanschette um 180° gedreht werden muss (siehe auch Figur 9 im Vergleich mit Figur 11). Entscheidend für das erfindungsgemäße System ist, dass die Stützfeder in verschiedenen Größen, aber auch in verschiedenen Federstärken (Härten) hergestellt werden kann. Die unterschiedlichen Federstärken können durch unterschiedliche Materialien, unterschiedliche Materialstärken oder auch durch gezielte Verstärkungen (eingebettete Stäbe, Rippen etc.) ausgebildet werden. Auf diese Weise kann beispielweise nach erfolgter Anpassung von Fuß- und Unterschenkelmanschette eine Auswahl verschiedener Stützfedern am Patienten getestet werden. Dabei können die verschiedenen Tragweisen (medial v. lateral, ventral v. dorsal) wie auch unterschiedliche Härtegrade (Federstärken) unmittelbar miteinander verglichen werden. Patient und Therapeut können auf diese Weise das Gangbild des Patienten vergleichen und so die optimale Orthese gestalten.

Ein weiterer wesentlicher Gesichtspunkt der vorliegenden Erfindung ist die Verklemmung und Verschraubung von Unterschenkelmanschette und Stützfeder. Bei der ansonsten üblichen starren Verschraubung kommt es bei der Bewegung zu einer Relativbewegung zwischen Unterschenkel und der Unterschenkelmanschette, so dass die Ausbildung von Druckstellen und Abschürfungen möglich ist. Das erfindungsgemäße System enthält an dieser Stelle ein Verbindungselement welches durch Verklemmung und Verschraubung wirkt. Dabei ist die Mutter unidirektional schwimmend gelagert. Die Lagerung enthält hierzu ein Elastomer welches zu einer gelenkartigen Verbindung führt.
Der zentrale Bestandteil dieses Verbindungselementes ist bevorzugt X-förmig ausgebildet ("Starfish"). Die Unterschenkelmanschette enthält in einer bevorzugten Ausführungsform der Erfindung eine gleichfalls X-förmige Ausnehmung, die zur Aufnahme dieses Verbindungselements ("Starfish") vorgesehen ist. Das Verbindungselement ("Starfish") besteht aus einem festen Material und enthält eine Ausnehmung. In die Ausnehmung wird formschlüssig ein Verbindungsstück, welches aus einem Elastomer gefertigt ist. Durch dieses elastische Verbindungsstück werden die oben genannten Relativbewegungen minimiert. Dabei ist das Elastomer in verschiedenen Elastizitätsgraden herstellbar, so dass auch die notwendige Dämpfung der Relativbewegung durch Tests am Patienten ausprobiert werden kann.

Gegenstand der vorliegenden Erfindung ist auch eine Unterschenkelorthese, enthaltend eine thermisch ausgeformte Sohle montiert durch reversiblen Formschluss
mit einer oder mehreren vorgeformten Aufnahmen für Verriegelungselemente und einer sandalenförmigen Fußmanschette mit Polsterung und Fixiereinrichtungen,
eine Unterschenkelmanschette mit einer oder mehreren Fixiereinrichtung zur Befestigung am Unterschenkel, einer Polsterung sowie einer Aufnahmevorrichtung für ein oder mehrere Stützfeder, eine Stützfeder mit einem oberen flachen Teil sowie einem unteren halbrund ausgeformten Teil, wobei das halbrund ausgeformte Teil eine Ausnehmung zur Aufnahme der Sohle ausbildet,
wobei die Stützfeder mit der Unterschenkelmanschette reversibel verbunden ist,
wobei die Stützfeder an der Fußmanschette dorsal/medial, dorsal/ lateral, ventral/medial oder ventral/lateral befestigt ist.

Gegenstand der vorliegenden Erfindung ist ferner ein Kit zur individuellen Adaption einer Unterschenkelorthese gemäß, enthaltend
einer Auswahl thermisch ausgeformter Sohlen montierbar durch reversiblen Formschluss mit einer oder mehreren vorgeformten Aufnahmen für Verriegelungselemente,
einer Auswahl sandalenförmigen Fußmanschetten mit Polsterung und Fixiereinrichtungen,
einer Auswahl von Unterschenkelmanschetten mit einer oder mehreren Fixiereinrichtungen zur Befestigung am Unterschenkel, einer Polsterung sowie einer Aufnahmevorrichtung für ein oder mehrere Stützfedern,
einer Auswahl verschiedener Verbindungselemente für die reversible Verbindung von Sohle und Stützfeder mit verschiedenen Elastizitätsgraden, einer Auswahl von Stützfedern mit einem oberen flachen Teil sowie einem unteren halbrund ausgeformten Teil, wobei das halbrund ausgeformte Teil eine Ausnehmung zur Aufnahme der Sohle ausbildet,
wobei die Stützfeder mit der Unterschenkelmanschette reversibel verbindbar ist und wobei
die Stützfeder an der Fußmanschette dorsal/medial, dorsal/ lateral, ventral/medial oder ventral/lateral zu befestigen ist.

Dadurch, dass das erfindungsgemäße modulare Orthesensystem nur wenige variable Bauteile aufweist, kann dem behandelnden Therapeuten und beratenden Orthopädietechniker ein derartiges Kit zur Verfügung gestellt werden, welches jeweils eine Serie der variablen Bauteile mit unterschiedlichen Eigenschaften enthält. Nach Auswahl der Fuß- und Unterschenkelmanschette (ggf. nach individueller Anfertigung der Fußmanschette und Unterschenkelmanschette, beispielsweise mittels eines Gipsabdruckes) kann dem Therapeuten und beratenden Orthopädietechnikern eine Reihe verschiedener vorgefertigter Sohlen (mit unterschiedlichen Absatzhöhen bzw. unterschiedlichen Härtegraden), unterschiedlichen Stützfedern (für unterschiedliche Trageweisen und unterschiedlichen Längen) und ggf. verschiedenen Verbindungselementen mit verschiedenen Elastizitätsgraden zur Verfügung gestellt werden.

Der Orthopädietechniker oder der Therapeut kann mit Hilfe des Kits für jeden Patienten die Anpassung individuell vornehmen. Von besonderer Bedeutung ist dabei die Auswahl der korrekt ausgeformten Sohle und der Stützfeder. Hier kann der Therapeut zusammen mit dem Patienten unterschiedliche Federstärken testen, aber auch die Position der Stützfeder (dorsal vs. ventral, medial vs. lateral) ausprobieren. Auf diese Weise kann die Anpassung der Orthese an den Patienten auf einfache Weise durchgeführt werden.

So kann beispielsweise eine Videoaufzeichnung des Gangbildes des jeweiligen Patienten beispielsweise auf einem Laufband aufgenommen werden. Die verschiedenen Laufbilder können dann nebeneinander dargestellt werden und erlauben dem Therapeuten die Auswahl des optimalen Bewegungsbildes, natürlich unter Berücksichtigung des individuellen Tragegefühls des Patienten. Ist die Auswahl für Grösse, Federstärke und Trageposition optimiert, kann der Therapeut die einzelnen Komponenten für den Patienten zusammenstellen. Auf diese Weise ist eine einfache Anpassung an den Patienten möglich.

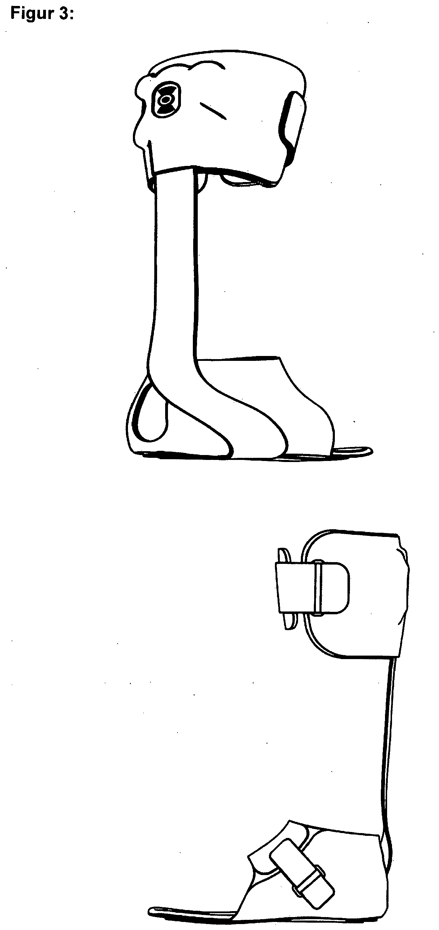

Die Erfindung wird durch die folgenden Abbildungen beispielhaft weiter erläutert.

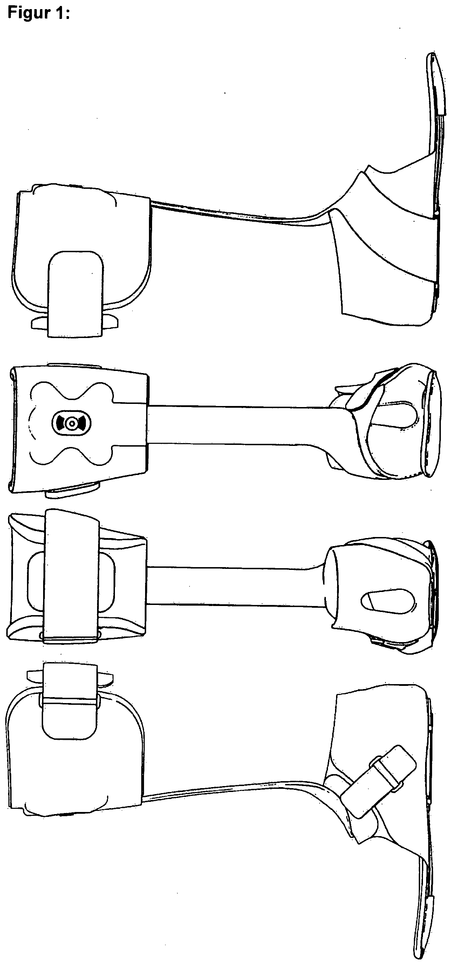

- Figur 1: zeigt eine Übersicht über ein erfindungsgemäßes Orthesensystem aus vier verschiedenen Blickwinkeln.
- Figur 2: zeigt den modularen Aufbau des erfindungsgemäßen Orthesensystems enthaltend folgende Teile:
1: Sohle
2: Stützfeder
3: Verbindungselement (Starfish) mit Ausnehmung
4: Sohlenverriegelung
5: Afferenz verstärkende Sohlenverriegelung
6: Verbindungselement (Elastomer) für Starfish Ausnehmung
7: Schraube
8: Sandalenförmige Fußmanschette
9: Unterschenkelmanschette
10: Polsterung
11: Verschlusselement (Fixiereinrichtung)
12: Verschlusselement (Fixiereinrichtung) 13: Optional zusätzliches Schutzelement (Bumper) für Stützfeder
- Figur 3: zeigt ein erfindungsgemäßes Orthesensystem, bei dem die Stützfeder dorsal (achillessehnenseitig) angebracht ist aus zwei verschiedenen Blickwinkeln.

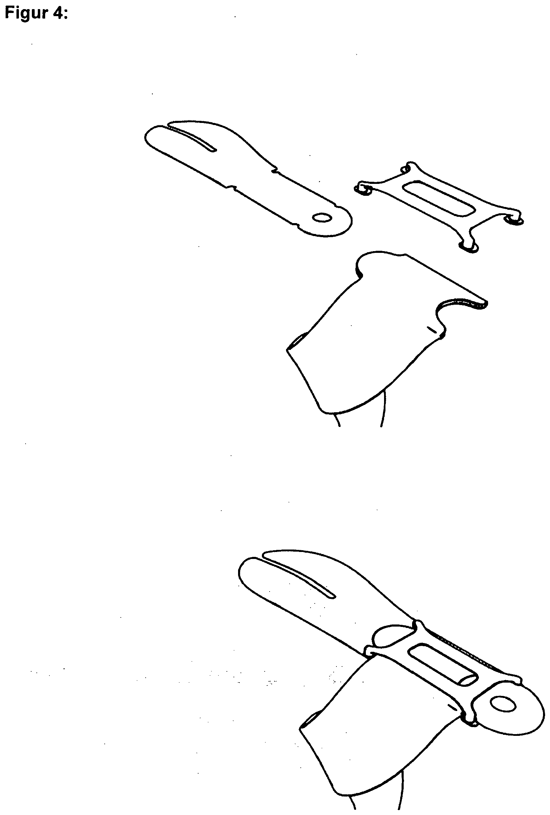

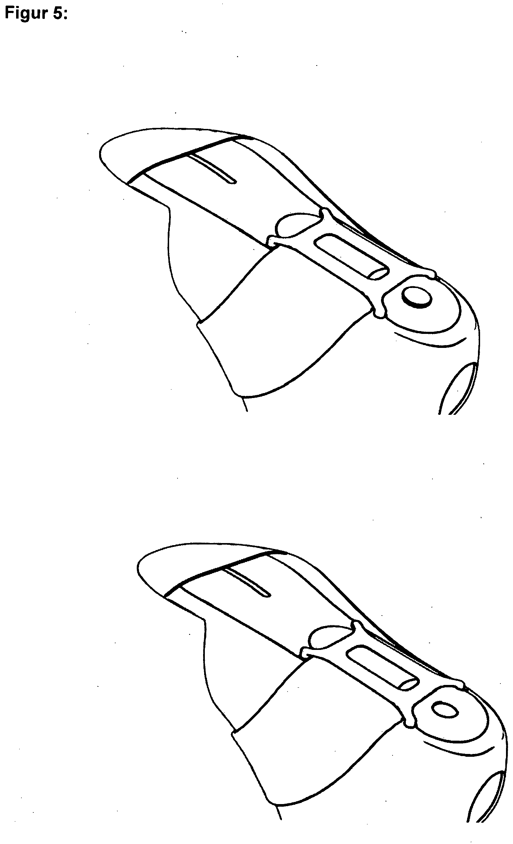

- Figuren 4 + 5: zeigen in einer Bildsequenz Details der Befestigung der Fußmanschette

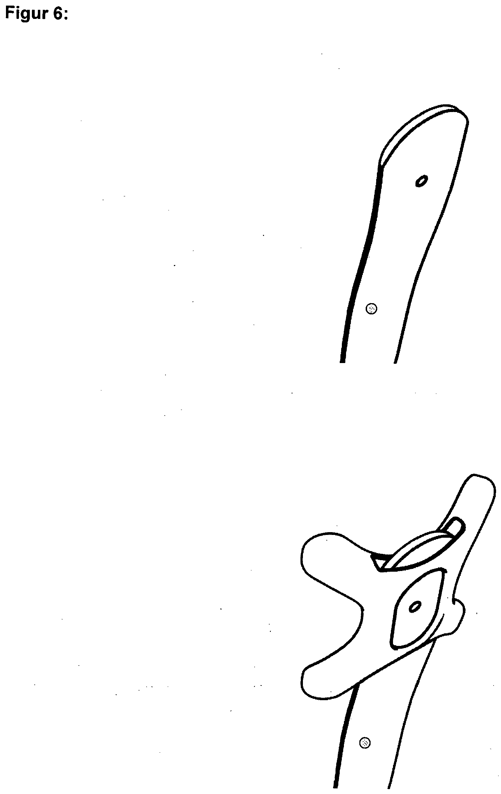

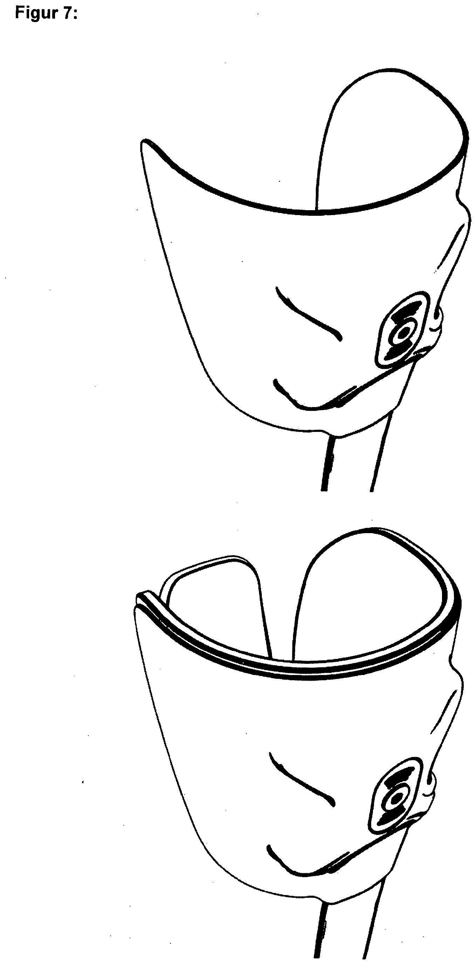

- Figuren 6 + 7: zeigen in einer Bildsequenz die Befestigung der Stützfeder an der Unterschenkelmanschette mittels erfindungsgemäßem Verbindungselement in X-förmiger Ausgestaltung (Starfish) inkl. Elastomer mit schwimmend gelagerter Schraube und Polsterung

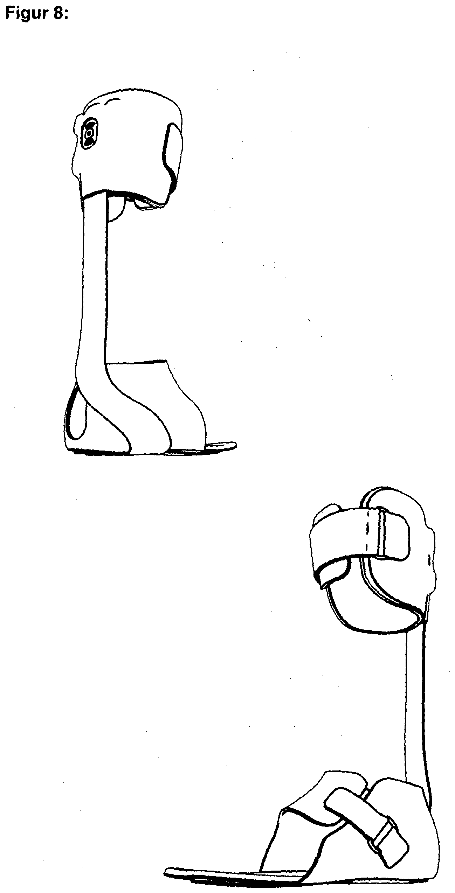

- Figuren 8 + 9: zeigen ein erfindungsgemäßes Orthesensystem mit dorsaler Befestigung der Unterschenkelmanschette

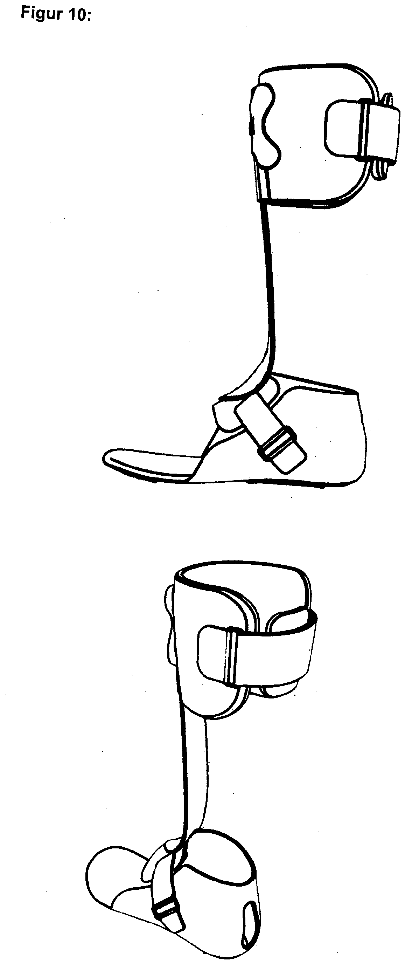

- Figur 10: zeigt ein erfindungsgemäßes Orthesensystem mit ventraler Befestigung der Unterschenkelmanschette

Die jeweiligen Polsterungen werden aus atmungsaktiven Materialien erstellt, z.B. aus waschbarem, wärme- und feuchtigkeitstransportierendem, luftdurchlässigen, klettfähigen 3D-Abstandsgewirke (z.B. Space Tex), die in der Waschmaschine beispielsweise bei 30° gewaschen werden können.

Die Sohle und insbesondere die Stützfeder werden unter Verwendung von Materialien zur Herstellung von federnden und energiespeichernden Bauteilen erstellt, wie z.B. Carbonfaser-Prepreg.

Ein besonderer Vorteil des erfindungsgemäßen Orthesensystems ist die Möglichkeit, die Orthese dynamisch anzupassen, insbesondere in der Wachstumsphase bei Kindern und Jugendlichen. Die einzelnen Bauteile und das Schutzelement (Bumper) können äusserlich unterschiedlich gestaltet werden, beispielsweise durch Aufdruck, Gravur oder ähnliches. Auf diese Weise können die Orthesen, insbesondere für Kinder und Jugendliche, gestalterisch individualisiert werden, so dass die Trageakzeptanz deutlich erhöht wird.

## Patentansprüche

1. Modulares Herstellungs- und Adaptionssystem für Orthesen, enthaltend eine thermisch ausgeformte Sohle (1) zur Montage durch reversiblen Formschluss mit einer oder mehreren vorgeformten Aufnahmen für Verriegelungselemente (4) und einer sandalenförmigen Fußmanschette (8) mit Polsterung und Fixiereinrichtungen,
eine Unterschenkelmanschette (9) mit einer oder mehreren Fixiereinrichtung zur Befestigung am Unterschenkel, einer Polsterung (10) sowie einer Aufnahmevorrichtung für eine oder mehrere Stützfeder, eine Stützfeder (2) mit einem oberen flachen Teil sowie einem unteren halbrund ausgeformten Teil, wobei das halbrund ausgeformte Teil eine Ausnehmung zur Aufnahme der Sohle ausbildet,
wobei die Stützfeder mit der Unterschenkelmanschette mittels eines Verbindungselementes (3) reversibel verbunden ist, **dadurch gekennzeichnet, dass**
die Stützfeder an der Fußmanschette dorsal/medial, dorsal/lateral, ventral/medial oder ventral/lateral befestigt sein kann.

2. Modulares Herstellungs- und Adaptionssystem für Orthesen gemäß Anspruch 1, **dadurch gekennzeichnet, dass** die reversible Verbindung von Unterschenkelmanschette und Stützfeder durch Verschraubung mit einem unidirektional schwimmend gelagerten Verbindungselement erfolgt.

3. Modulares Herstellungs- und Adaptionssystem, gemäß Anspruch 1, **dadurch gekennzeichnet, dass** die reversible Verbindung von Sohle und Stützfeder durch Klemmung unter Nutzung von Riegelelementen erfolgt.

4. Modulares Herstellungs- und Adaptionssystem, gemäß Anspruch 1 und 3, **dadurch gekennzeichnet, dass** ein Verriegelungselement für die reversible Verbindung von Sohle und Stützfeder sich zentral unter der Ferse befindet und elastisch, aber weniger elastisch als die Polsterung der Fußmanschette ausgestaltet ist.

5. Modulares Herstellungs- und Adaptionssystem für Orthesen gemäß Anspruch 1, **dadurch gekennzeichnet, dass** Sohle und/oder Stützfeder und/oder Aufnahmevorrichtung aus vorimprägnierten Faserträgermaterialien, bevorzugt PrePreg in Carbon, gefertigt sind.

6. Modulares Herstellungs- und Adaptionssystem für Orthesen gemäß Anspruch 1, **dadurch gekennzeichnet, dass** Unterschenkelmanschette und/oder Fußmanschette aus thermoplastischem Werkstoff gefertigt sind.

7. Modulares Herstellungs- und Adaptionssystem für Orthesen gemäß Anspruch 1, **dadurch gekennzeichnet, dass** die Stützfeder formschlüssig mit einem weichen Schutzelement bezogen ist.

8. Modulares Herstellungs- und Adaptionssystem, gemäß Anspruch 1, **dadurch gekennzeichnet, dass** die Polsterungen von Unterschenkelmanschette und/oder Fußmanschette aus waschbarem, wärme- und feuchtigkeitstransportierendem, luftdurchlässigen, klettfähigen 3D-Abstandsgewirke gefertigt sind.

9. Unterschenkelorthese, enthaltend eine thermisch ausgeformte Sohle (1) montiert durch reversiblen Formschluss mit einer oder mehreren vorgeformten Aufnahmen für Verriegelungselemente (4) und einer sandalenförmigen Fußmanschette (8) mit Polsterung und Fixiereinrichtungen, eine Unterschenkelmanschette (9) mit einer oder mehreren Fixiereinrichtung zur Befestigung am Unterschenkel, einer Polsterung sowie einer Aufnahmevorrichtung für eine oder mehrere Stützfeder, eine Stützfeder (2) mit einem oberen flachen Teil sowie einem unteren halbrund ausgeformten Teil, wobei das halbrund ausgeformte Teil eine Ausnehmung zur Aufnahme der Sohle ausbildet,
wobei die Stützfeder mit der Unterschenkelmanschette reversibel verbunden ist, **dadurch gekennzeichnet, dass** die Stützfeder an der Fußmanschette dorsal/medial, dorsal/ lateral, ventral/medial oder ventral/lateral befestigt ist.

10. Unterschenkelorthese gemäß Anspruch 9, **dadurch gekennzeichnet, dass** die reversible Verbindung von Unterschenkelmanschette und Stützfeder durch Verschraubung mit einem unidirektional schwimmend gelagerten Verbindungselement erfolgt.

11. Unterschenkelorthese gemäß Anspruch 9, **dadurch gekennzeichnet, dass** die reversible Verbindung von Sohle und Stützfeder durch Klemmung unter Nutzung von Riegelelementen erfolgt.

12. Unterschenkelorthese gemäß Anspruch 9 und 11, **dadurch gekennzeichnet, dass** ein Verriegelungselement für die reversible Verbindung von Sohle und Stützfeder sich zentral unter der Ferse befindet und elastisch, aber weniger elastisch als die Polsterung der Fußmanschette ausgestaltet ist.

13. Kit zur individuellen Adaption einer Unterschenkelorthese gemäß Anspruch 9, enthaltend
einer Auswahl thermisch ausgeformter Sohlen montierbar durch reversiblen Formschluss mit einer oder mehreren vorgeformten Aufnahmen für Verriegelungselemente,
einer Auswahl sandalenförmigen Fußmanschetten mit Polsterung und Fixiereinrichtungen,
einer Auswahl von Unterschenkelmanschetten mit einer oder mehreren Fixiereinrichtungen zur Befestigung am Unterschenkel, einer Polsterung sowie einer Aufnahmevorrichtung für ein oder mehrere Stützfedern, einer Auswahl verschiedener Verbindungselemente für die reversible Verbindung von Sohle und Stützfeder mit verschiedenen Elastizitätsgraden,
einer Auswahl von Stützfedern mit einem oberen flachen Teil sowie einem unteren halbrund ausgeformten Teil, wobei das halbrund ausgeformte Teil eine Ausnehmung zur Aufnahme der Sohle ausbildet, wobei die Stützfeder mit der Unterschenkelmanschette reversibel verbindbar ist und wobei
die Stützfeder an der Fußmanschette dorsal/medial, dorsal/ lateral, ventral/medial oder ventral/lateral zu befestigen ist.

## Claims

1. A modular manufacture and adaptation system for orthoses, containing a thermally molded sole (1) for mounting by reversible form-fit with one or more preformed receptacles for locking elements (4) and a sandal-shaped foot cuff (8) with padding and fixing devices, a lower leg cuff (9) with one or more fixing devices for attachment to the lower leg, a padding (10) and a receiving device for one or more support springs, a support spring (2) having an upper flat portion and a lower semi-circular shaped portion, the semi-circular shaped portion forming a recess for receiving the sole, the support spring being reversibly connected to the lower leg cuff by means of a joining element (3), **characterized by that** the support spring can be attached at the foot cuff in a dorsal/medial, dorsal/lateral, ventral/medial or ventral/lateral manner.

2. The modular manufacture and adaptation system for orthoses according to claim 1, **characterized by that** the reversible connection of lower leg cuff and support spring occurs by screw connection with a unidirectionally floating joining element.

3. The modular manufacture and adaptation system according to claim 1, **characterized by that** the reversible connection of sole and support spring occurs by clamping using locking elements.

4. The modular manufacture and adaptation system according to claim 1 and 3, **characterized by that** a locking element for the reversible connection of sole and support spring is located centrally below the heel and is resiliently configured, however less resilient than the padding of the foot cuff.

5. The modular manufacture and adaptation system for orthoses according to claim 1, **characterized by that** the sole and/or the support spring and/or the receiving device are made of pre-impregnated fibrous carrier materials, preferably pre-preg in carbon.

6. The modular manufacture and adaptation system for orthoses according to claim 1, **characterized by that** the lower leg cuff and/or the foot cuff are made of a thermoplastic material.

7. The modular manufacture and adaptation system for orthoses according to claim 1, **characterized by that** the support spring is covered in a form-fit manner with a soft protective element.

8. The modular manufacture and adaptation system according to claim 1, **characterized by that** the paddings of the lower leg cuff and/or the foot cuff are made of a washable, heat- and moisture-transporting, air-permeable, hook-and-loop-connectable 3D spacer fabric.

9. A lower leg orthosis, containing a thermally molded sole (1) mounted by reversible form-fit with one or more preformed receptacles for locking elements (4) and a sandal-shaped foot cuff (8) with padding and fixing devices, a lower leg cuff (9) with one or more fixing devices for attachment at the lower leg, a padding and a receiving device for one or more support springs, a support spring (2) having an upper flat portion and a lower semi-circular shaped portion, the semi-circular shaped portion forming a recess for receiving the sole, the support spring being reversibly connected to the lower leg cuff, **characterized by that** the support spring is attached at the foot cuff in a dorsal/medial, dorsal/lateral, ventral/medial or ventral/lateral manner.

10. The lower leg orthosis according to claim 9, **characterized by that** the reversible connection of lower leg cuff and support spring occurs by screw connection with a unidirectionally floating joining element.

11. The lower leg orthosis according to claim 9, **characterized by that** the reversible connection of sole and support spring occurs by clamping using locking elements.

12. The lower leg orthosis according to claim 9 and 11, **characterized by that** a locking element for the reversible connection of sole and support spring is located centrally below the heel and is resiliently configured, however less resilient than the padding of the foot cuff.

13. A kit for individual adaptation of a lower leg orthosis according to claim 9, containing
a selection of thermally molded soles to be mounted by reversible form-fit with one or more preformed receptacles for locking elements,
a selection of sandal-shaped foot cuffs with padding and fixing devices,
a selection of lower leg cuffs with one or more fixing devices for attachment at the lower leg, a padding and a receiving device for one or more support springs,
a selection of different joining elements for the reversible connection of sole and support spring with different degrees of elasticity,
a selection of support springs with an upper flat portion and a lower semi-circular shaped portion, the semi-circular shaped portion forming a recess for receiving the sole, the support spring being reversibly connected to the lower leg cuff, and the support spring being attachable at the foot cuff in a dorsal/medial, dorsal/lateral, ventral/medial or ventral/lateral manner.

## Revendications

1. Système modulaire de fabrication et d'adaptation d'orthèses, contenant une semelle (1) thermiquement moulée pour montage par engagement positif réversible avec un ou plusieurs logements pré-formés pour des éléments de verrouillage (4) et un collier de pied (8) en forme de sandale avec une matelassure et des dispositifs de fixation, un collier d'extrémité inférieure (9) avec un ou plusieurs dispositifs de fixation pour l'attache à l'extrémité inférieure, une matelassure (10) et un dispositif de logement pour un ou plusieurs ressorts de soutien, un ressort de soutien (2) comportant une partie supérieure plate et une partie inférieure en forme semi-circulaire, la partie inférieure en forme semi-circulaire formant un évidement pour loger la semelle, le ressort de soutien étant lié de façon réversible au collier d'extrémité inférieure au moyen d'un élément de liaison (3), **caractérisé en ce que** le ressort de soutien peut être attaché au collier de pied de façon dorsale/médiale, dorsale/latérale, ventrale/médiale ou ventrale/latérale.

2. Système modulaire de fabrication et d'adaptation d'orthèses selon la revendication 1, **caractérisé en ce que** la liaison réversible du collier d'extrémité inférieure et du ressort de soutien se fait par vissage avec un élément de liaison supporté de façon unidirectionnellement flottante.

3. Système modulaire de fabrication et d'adaptation selon la revendication 1, **caractérisé en ce que** la liaison réversible de la semelle et du ressort de soutien se fait par clipsage en utilisant des éléments de verrouillage.

4. Système modulaire de fabrication et d'adaptation selon la revendication 1 et 3, **caractérisé en ce qu**'un élément de verrouillage pour la liaison réversible de la semelle et du ressort de soutien se trouve centralement au-dessous du talon et est configuré de façon élastique, mais moins élastique que la matelassure du collier de pied.

5. Système modulaire de fabrication et d'adaptation d'orthèses selon la revendication 1, **caractérisé en ce que** la semelle et/ou le ressort de soutien et/ou le dispositif de logement sont en des matériaux de support fibreux pré-imprégnés, de préférence en prépreg en carbone.

6. Système modulaire de fabrication et d'adaptation d'orthèses selon la revendication 1, **caractérisé en ce que** le collier d'extrémité inférieure et/ou le collier de pied sont en un matériau thermoplastique.

7. Système modulaire de fabrication et d'adaptation d'orthèses selon la revendication 1, **caractérisé en ce que** le ressort de soutien est recouvert par engagement positif d'un élément de protection souple.

8. Système modulaire de fabrication et d'adaptation selon la revendication 1, **caractérisé en ce que** les matelassures du collier d'extrémité inférieure et/ou du collier de pied sont en un tricot d'écartement tridimensionnel lavable, transportant la chaleur et l'humidité, perméable à l'air, pour la liaison du type de bouclettes et crochets.

9. Orthèse d'extrémité inférieure, comportant une semelle (1) thermiquement moulée pour montage par engagement positif réversible avec un ou plusieurs logements pré-formés pour des éléments de verrouillage (4) et un collier de pied (8) en forme de sandale avec une matelassure et des dispositifs de fixation, un collier d'extrémité inférieure (9) avec un ou plusieurs dispositifs de fixation pour l'attache à l'extrémité inférieure, une matelassure et un dispositif de logement pour un ou plusieurs ressorts de soutien, un ressort de soutien (2) comportant une partie supérieure plate et une partie inférieure en forme semi-circulaire, la partie en forme semi-circulaire formant un évidement pour loger la semelle, le ressort de soutien étant lié de façon réversible au collier d'extrémité inférieure, **caractérisé en ce que** le ressort de soutien peut être attaché au collier de pied de façon dorsale/médiale, dorsale/latérale, ventrale/médiale ou ventrale/latérale.

10. Orthèse d'extrémité inférieure selon la revendication 9, **caractérisée en ce que** la liaison réversible du collier d'extrémité inférieure et du ressort de soutien se fait par vissage avec un élément de liaison supporté de façon unidirectionnellement flottante.

11. Orthèse d'extrémité inférieure selon la revendication 9, **caractérisée en ce que** la liaison réversible de la semelle et du ressort de soutien se fait par clipsage en utilisant des éléments de verrouillage.

12. Orthèse d'extrémité inférieure selon la revendication 9 et 11, **caractérisée en ce qu**'un élément de verrouillage pour la liaison réversible de la semelle et du ressort de soutien se trouve centralement au-dessous du talon et est configuré de façon élastique, mais moins élastique que la matelassure du collier de pied.

13. Jeu pour l'adaptation individuelle d'une orthèse d'extrémité inférieure selon la revendication 9, comportant
une sélection de semelles thermiquement moulées pour le montage par engagement positif réversible avec un ou plusieurs logements pré-formés pour des éléments de verrouillage,
une sélection de colliers de pied en forme de sandale avec une matelassure et des dispositifs de fixation,
une sélection de colliers d'extrémité inférieure avec un ou plusieurs dispositifs de fixation pour l'attache à l'extrémité inférieure, une matelassure et un dispositif de logement pour un ou plusieurs ressorts de soutien,
une sélection d'éléments de liaison différents pour la liaison réversible de la semelle et du ressort de soutien avec des degrés d'élasticité différents,
une sélection de ressorts de soutien comportant une partie supérieure plate et une partie inférieure en forme semi-circulaire, la partie en forme semi-circulaire formant un évidement pour loger la semelle, le ressort de soutien étant lié de façon réversible au collier d'extrémité inférieure, et le ressort de soutien pouvant être attaché au collier de pied de façon dorsale/médiale, dorsale/latérale, ventrale/médiale ou ventrale/latérale.
